# EUROPEAN PATENT SPECIFICATION

(11) **EP 1 091 750 B1**
(45) Date of publication and mention of the grant of the patent: **02.09.2009**
(21) Application number: 99909670.4
(22) Date of filing: 26.02.1999
(51) Int. Cl.: A61K 38/13, A61K 47/20, A61P 37/06, A61P 25/28

(54) **CEREBROSPINAL AND VASCULAR PHARMACEUTICAL COMPOSITION AND PROCESS FOR PREPARING THE SAME**
CEREBROSPINAL UND VASKULÄR WIRKSAME PHARMAZEUTISCHE ZUBEREITUNG UND VERFAHREN ZU IHRER HERSTELLUNG
COMPOSITION PHARMACEUTIQUE CEREBROSPINALE ET VASCULAIRE ET PROCEDE DE PREPARATION DE LADITE COMPOSITION

(43) Date of publication of application: 18.04.2001
(73) Proprietor: Maas BiolAB, LLC, Albuquerque, NM 87122 (US)
(72) Inventor: Keep, Marcus, Albuquerque, New Mexico 87122 (US); Elmer, Eskil, 226 47 Lund (SE)
(74) Representative: Grünecker, Kinkeldey, Stockmair & Schwanhäusser Anwaltssozietät
(86) International application number: PCT/US1999/004359
(87) International publication number: WO 2000/050058

(56) References cited:
- US-A- 4 649 047
- US-A- 5 807 820
- US-A- 5 811 410
- US-A- 5 827 834
- ELZINGA L W ET AL: "The effect of dimethyl sulfoxide on the absorption of cyclosporine in rats." TRANSPLANTATION. FEB 1989, vol. 47, no. 2, February 1989 (1989-02), pages 394-395, XP009043582 ISSN: 0041-1337
- SHIMIZU SHIGETOSHI ET AL: "Dimethylsulfoxide (DMSO) treatment reduces infarction volume after permanent focal cerebral ischemia in rats" NEUROSCIENCE LETTERS, vol. 239, no. 2-3, 19 December 1997 (1997-12-19), pages 125-127, XP002316700 ISSN: 0304-3940
- BROADWELL R D ET AL: "Morphologic effect of dimethyl sulfoxide on the blood-brain barrier." SCIENCE. 9 JUL 1982, vol. 217, no. 4555, 9 July 1982 (1982-07-09), pages 164-166, XP009043581 ISSN: 0036-8075

## Description

### Field of the Invention:

This invention relates to the use of pharmaceutical compositions that facilitate the administration of cyclosporins, particularly into cerebrospinal and vascular fluid spaces.

### Background Art

### Cyclosporin A and derivatives.

Cyclosporin A is an immunosuppressive drug. The above mentioned treatment medication has already been described, in United States Pat. No. 4,117,118 and numerous patents since, which relate to its production, formulation and immunosuppressive properties.

Cyclosporin A is a product of the fungus Tolypocladium Inflatum Gams. It is a cyclic poly-amino acid molecule, consisting of 11 amino acids. One of the amino acids is unique for cyclosporin A, a B-hydroxyamino acid called butenyl-methyl-threonin (MeBmt). The molecular weight is 1202.6 and the chemical composition is C₆₂H₁₁₁N₁₁O₁₂·

The molecule is highly lipophilic, and therefore virtually insoluble in water. The drug is transported in the blood within red blood cells to about 58%, and the remaining approximately 10-20% in leukocytes, and 33% bound to plasma proteins. In the plasma cyclosporin A is bound to high-density lipoprotein, low-density lipoproteins, very-low density lipoproteins and a small fraction to albumin. A very small fraction is free in plasma.

The drug undergoes extensive metabolism, mainly in the liver by the cytochrome P450 system. There are at least 30 known metabolites of cyclosporin A, with various chemical modifications, such as hydroxylation, demethylation, oxidation and epoxide formations. There are a number of variants of cyclosporin A, differing for example in one amino acid, which have similar pharmacological properties. Under normal conditions, cyclosporin A and its metabolites do not pass the blood-brain barrier. When the glycoprotein-p transporter is poisoned, or the blood-brain barrier is disrupted, cyclosporin is able to cross it and come into contact with neurons. Several analogs of cyclosporin are able to readily cross the blood-brain barrier. Several analogs of cyclosporin are not immunosuppressants. There is a subset of analogs of cyclosporin that both readily cross the blood-brain barrier and are not immunosuppressants.

The family of cyclosporins includes cyclosporin A, cyclosporin C, cyclosporin D, and cyclosporin G. Some known metabolites of cyclosporin A include the following : (according to Hawk's Cay nomenclature) AM1, AM9, AM1c, AM4N, AM19, AM1c9, AM1c4N9, AM1A, AM1A4N, AM1Ac, AM1AL, AM11d, AM69, AM4N9, AM14N, AM14N9, AM4N69, AM99N, Dihydro-CsA, Dihydro-CsC, Dihydro-CsD, Dihydro-CsG, M 17, AM1c-GLC, sulphate conjugate of cyclosporin, BH11a, BH15a, B, G, E, (and with come overlap with the Hawk's above, according to Maurer nomenclature) M1, M2, M3, M4, M5, M6, M7, M8, M9, M10, M11, M12, M13, M14, M15, M16, M 17, M 18, M 19, M20, M21, M22, M23, M24, M25, M26, MUNDF1 and MeBMT. Some metabolites of cyclosporin G include GM1, GM9, GM4N, GM1c, GM1c9, and GM19. Modified cyclosporins include modified C-9 amino acid analogs, modified 8-amino acid analogs, modified 6-position analogs containing MeAla or MeAbu residue, and SDZ 209-313, SDZ-205-549, SDZ-033-243, and SDZ-PSC-833.

This entire family of cyclosporins, including cyclosporin A, all derivatives, variants, amino acid variants, metabolites, including variations of mono-, di- and trihydroxylates, N-demethylates, aldehydes, carboxylates, conjugates, sulphates, glucuronides, intramolecular cyclizations and those without a cyclic structure as well as shorter peptides and amino acids and their derivatives and salts with or without immunosuppressive properties and whether able to cross the blood brain barrier or not will hereinafter be referred to as cyclosporin or cyclosporins.

Cyclosporins are highly lipophilic and virtually insoluble in water. They require an emulsifier to remain in aqueous phase, such as cremophore or Labrafil. One problem is that these emulsifiers are anaphylactic, and are neurotoxic. There is a need for an improved formulation for intravenous and oral use that is neither anaphylactic nor neurotoxic.

Therefore our aim was to use a unique cerebrospinal pharmaceutical composition with cyclosporin as the active ingredient that does not contain dangerous neurotoxic emulsifiers and irritants found in vascular and oral formulations.

Now we have found that if cyclosporin is dissolved in DMSO (dimethyl sulfoxide), the neurotoxic and irritant effects on the nervous system are not seen. Further we have found that not only is it a unique new cerebrospinal fluid composition with great safety and free from neurotoxicity, but that it is also an improve formulation for intravascular and oral administration too.

Dimethyl sulfoxide is an industrial solvent derived from wood. It is biocompatible, but approved in only one condition for human use, that of interstitial cystitis, a completely unrelated bladder condition. DMSO may by itself be useful for other conditions such as scleroderma, arthritis, mental illness, and brain trauma, but is not accepted into general medical use for these conditions.
US 4, 649, 047, and US 5, 807, 820 disclose pharmaceutical compositions comprising cyclosporin and DMSO. Elzinga et.al (1989) discloses the effect of DMSO on cyclosporin absorption in rats.

### The Instant Invention.

Cyclosporins are highly lipid soluble. There are well known to the art a number of mixtures for oral and intravenous administration, usually involving a carrier solution of lipid in water emulsion, micro-emulsion or nano-emulsion or particles. For intravenous administration this lipid in water emulsion is designed to allow administration in fluid phase mixed with salt-water solution to allow easy flow without precipitation of cyclosporin in the administering tubing or the blood. This has been acceptable in the past for simple intravenous (or oral) administration at the relatively low doses needed to treat immune rejection in transplantation or autoimmmune disease.

It has been recently discovered that cyclosporin is neuroprotective when it comes into contact with neurons. Since the drugs are also systemically immunosuppressive, it would be desirable to administer them selectively into the brain or the cerebrospinal fluid around the brain. Nonsystemic local CSF administration of cyclosporin would reduce systemic immune suppression, and increase brain and spine neuron exposure compared to systemic administration, both very desirable goals in patients in need of neuroprotection but not in need of immunosuppression. Especially patients that need long term treatment neuroprotection (such as those with amyotrophic lateral sclerosis, Parkinson's disease and Alzheimer's disease), it is desirable to avoid the complication, or undesirable side effect of lifelong systemic immunosuppression.

Lipids emulsifiers are used in Sandimmune (Novartis' formulation for cyclosporin) such as a modified castor oil derivative (Labrafil and cremophore). These castor oil derivatives have been themselves found to be directly neurotoxic, and likely to be the cause of the reported cases of neurologic problems of encephalopathy, and seizures in transplant patients.

Other formulations use less neurotoxic lipid formulations such as toceraphols. However lipids injected into the cerebrospinal fluid have been reported to cause severe complications of archnoiditis or inflammation around the spinal cord-such as the discontinued oil based Pantopaque intrathecal X-ray contrast used in spinal myelograms. Injecting lipids, even in micro or nano-emulsions into the cerebrospinal spaces would likely cause layering of the lipid, since the long column of fluid in the spine contains slow moving CSF, which is a very different milieu than the rapidly flowing and mixing turbulent arterial and venous blood. The layering of lipid and drug in the cerebrospinal fluid column would result in undesirable cyclosporin over or underdosage, depending on location.

In addition, ethanol is a commonly used solvent for cyclosporin used in Sandimmune. and SangCya (Sangstat's cyclosporin formulation). Ethanol is a known neurotoxin, and is used for neurolysis or destruction of nerve roots by injection into the brain or cerebrospinal fluid spaces.

It is now found to be desirable to inject cyclosporin into the fluid contained within the brain, and in which the brain floats, the cerebrospinal fluid.

All previous formulations for cyclosporin have utilized a lipid emulsifier to obtain solubility. It would not be expected by a person skilled in the art that a completely different class of compound other than a lipid emulsifier, such as the industrial solvent DMSO would turn out to be suitable for intracerebrospinal administration. Further they would not expect that the use of an industrial solvent would be an improvement over existing intravascular and oral cyclosporin compositions.

This formulation for administering cyclosporin into the CSF is the use of dimethylsulfoxide (DMSO) as the carrier medium or solvent. Cyclosporin is very soluble in DMSO. Unlike castor oil derivatives or ethanol, DMSO is not neurotoxic. It is highly biocompatable and is actually a known neuroprotectant in its own right. It is possible to safely administer DMSO. DMSO prevents precipitation of cyclosporin even when diluted in the CSF.

In addition DMSO easily crosses tissues barriers in its role as a solvent and carries in with it drugs dissolved in it. This is an advantage because it opens the blood-brain barrier and CSF-brain barrier to facilitate cyclosporin penetration into the brain.

The two main methods for infusing the formulation will be via the cerebral ventricles and the thecal sac. A catheter placed through a scalp incision, a skull twist drill hole and the brain, will access the cerebral ventricles. If a short-term infusion of several days is desired the catheter would be remain externalized and then removed. If a long term infusion over months or years were desired, the skin would be closed over the catheter and an attached refillable reservoir or drug pump.

Thecal sac infusion is typically by lumbar puncture in the lumbar spine (or less often at the top of the spine at the base of the skull).The spinal puncture needle is placed through the skin of the back and pierces the dura (thecal sac) which contains the CSF in which the spinal cord is floating. Once in, formulary drug can be infused into the CSF in one session via the needle, or over a week through a soft catheter. For long term infusion over months or years, the soft catheter is connected under the skin to a refillable programmable pump that is usually implanted under he abdominal skin.

In addition to being a novel, unique, efficacious and safe formulation for intrathecal administration, this formulation is also superior for intravenous or intra-arterial administration. The absence of systemic neurotoxic castor oil derivatives will reduce the well-known neurological complications. The tissue penetrating capabilities of DMSO will facilitate penetration of the blood-brain barrier by cyclosporin to come into contact with neurons to make it work better.

### Medicament and administration.

The formulary drug can be administration by the routes including oral, sublingual, buccal, nasal, inhalation, parenteral (including intraperitoneal, intraorgan, subcutaneous, intradermal, intramuscular, intra-articular, venous (central, hepatic or peripheral), lymphatic, cardiac, arterial, including selective or superselective cerebral arterial approach, retrograde perfusion through cerebral venous system, via catheter into the brain parenchyma or ventricles), direct exposure or under pressure onto or through the brain or spinal tissue, or any of the cerebrospinal fluid ventricles, injections into the subarachnoid, brain cisternal, subdural or epidural spaces, via brain cisterns or lumbar puncture, intra and peri-ocular instillation including application by injection around the eye, within the eyeball, its structures and layers, as well as via enteral, bowel, rectal, vaginal, urethral or bladder cisternal. Also for in *utero* and perinatal indications then injections into the maternal vasculature, or through or into maternal organs including the uterus, cervix and vagina, and into embryo, fetus, neonate and allied tissues and spaces such as the amniotic sac, the umbilical cord, the umbilical artery or veins and the placenta, with parenteral being the preferred route.

The formulary drug, containing cyclosporin dissolved in DMSO, for administration into the brain and related structures, spinal cord and related structures, ventricular system and cerebrospinal fluid spaces can be manufactured and distributed containing, aqueous and non-aqueous injection solutions, other pharmaceutically active compounds, additives including anti-oxidants, bacteriostats and solutes and sugars such as mannitol to make the formulary drug isotonic, hypotonic or hypertonic with the cerebrospinal fluid; and also aqueous and non-aqueous sterile suspensions. The formulary drug can be manufactured and distributed in unit-dose or multi-dose containers, such as sealed glass or plastic ampoules, vials, bottles and bags as a liquid, and in a dry state requiring the addition of DMSO.

The formulary drug for parenteral administration can be manufactured from cyclosporin, DMSO, aqueous sterile injection solutions, other pharmaceutically active compounds, additives including anti-oxidants, bacteriostats and solutes and sugars such as mannitol to make the formulary drug isotonic, hypotonic or hypertonic with the fluids of the recipient. The formulary drug can be manufactured and distributed in unit-dose or multi-dose containers, such as sealed glass or plastic ampoules, vials, bottles and bags as a liquid, and in a dry state requiring the addition of DMSO.

The formulations are used in patients who require neuroprotection from neurological diseases of acute to chronic nature including stroke, brain hemorrhage, brain and spine trauma, ionizing radiation, neurotoxicity to vestibulocochlear structures, retinal detachment and neurodegeneration including amyotrophic lateral sclerosis, Parkinson's and Alzheimer's.

The formulations are used in patients who require both neuroprotection from neurological disease and that their neuro-axis be immunocompromised, such as in neural transplantation, neural xenotransplantation, multiple sclerosis, HIV neuropathy and Guillain-Barré syndrome.

The formulations are used in patients who require that they be immunocompromised, such as in transplantation and autoimmune disease.

The formulations are used for topical application for patients who require immunocompromise of the skin for diseases such such as psoriasis.

The formulary drug generally contains from 0.1 to 90% of the treatment medication by weight of the total composition. Cerebrospinal doses between 5 mg and 5 gram per day are possible, with about 50-150 mg/day for chronic administration, and 100-1000 mg/day for acute administration being preferable. Amounts of from 0.0001 mg to 200 mg/kg, or preferably 0.001 to 50 mg/kg, of body weight per day for parenteral administration and 0.001 to 150 mg/kg orally, can be given. Nevertheless, it could be necessary to alter those dosage rates, depending on the condition, weight, and individual reaction of the subject to the treatment, and the mode in which the administration is carried out, and the stage of the disease process or interval of administration. It may thus be sometimes adequate to use less than the before stated minimum dose, while in other instances the upper limit must be surpassed to obtain therapeutic results.

### Examples:

### Example 1

Sterile Injectable Concentrate Formulary Drug with cyclosporin as active ingredient

| | | |
|---|---|---|
| Containing per ml: | | |
| Cyclosporin A | 200 | mg |
| DMSO | 800 | mg |

The formulary drug is made by dissolving 5 grams of cyclosporin into 20 grams dimethyl sulfoxide at room temperature. The solution thus obtained is made up to 25 ml with water. The solution is homogenized with stirring and filtered. The liquid is sterilized by radiation and then placed in a sealed container such as glass under inert gas atmosphere in doses of 1, 5 or 25 ml.

Sterile injectable concentrate formulary drug is administered, with or without dilution with for example isotonic saline, by infusion or by injection into cerebrospinal fluid spaces, brain, spine, vein or artery.

### Example 2

A person in need of acute brain or spinal neuroprotection from trauma or stroke has the composition of example 1 injected into the Cerebrospinal fluid of the ventricle of the brain through a burrhole in the skull, or into the cerebrospinal fluid of thecal sac via a lumbar puncture needle, or injected intravascularly.

### Example 3

A person in need of chronic brain or spinal neuroprotection from neurodegenerative disease such as Parkinson's, Alzheimer's or amyotrophic lateral sclerosis has the composition of example I injected periodically by a reservoir or pump into the cerebrospinal fluid of a brain ventricle through a burrhole in the skull, or into the cerebrospinal fluid of the thecal sac via a lumbar catheter connected to a reservoir and pump.

### Example 4

A person in need of neural immunosuppression for neural transplantation, neural xenotransplantation, or diseases with autoimmune components like multiple sclerosis, Guillain-Barré, has the composition of example 1 injected periodically by a reservoir or pump into the cerebrospinal fluid of a ventricle of the brain through a burrhole in the skull, or into the cerebrospinal fluid of the spinal thecal sac via a lumbar catheter connected to a reservoir and pump.

### Example 5

A person in need of systemic immunosuppression has intravenous injections or oral consumption of the compositions of examples 1.

### Industrial Applicability

From the above description it will be evident that the present invention provides improved compositions for the administration of cyclosporin. Additionally the present invention provides a completely new composition suitable for administration directly into the new target of delivery, the cerebrospinal fluid to directly treat diseases of the brain, which previously described compositions are not suitable for, because of the neurotoxicity of their solvents.

No precipitation was observed with an initial one month of testing of composition prepared according to example 1. Ampoules stored in darkness containing 5 ml stored at 0, 30, and 60 degrees C show neither discoloration nor precipitation.

Rats receiving intraventricular cerebrospinal infusions showed no neurotoxicity, seizures or untoward effects. In addition, rats receiving injections intravenously showed no ill effects.

## Claims

1. Use of a cyclosporin dissolved in DMSO in a pharmaceutically acceptable carrier for preparing a medicament for reducing or preventing neuronal damage and/or for causing immunosuppression.

2. The use of claim 1, wherein the medicament is prepared for injection into intraventricular fluid spaces, intrathecal cerebrospinal fluid spaces, intraocularly , intravestibularly, into the brain or into the spinal cord.

3. The use according to claim 2, wherein said medicament is prepared for administration by intra-ocular injection or by intravestibular injection into or adjacent to the brain or spinal cord into a human.

4. The use according to claim 1, wherein said medicament is prepared for administration by intraveneous, intra-arterial or intraparenchymal injection into a human.

5. The use according to claim 1, wherein said medicament is prepared for administration orally, rectally, nasally or dermally into a human.

6. The use according to claims 1 to 5, wherein said cyclosporin is selected from the group consisting of cyclosporin A, a functional derivative, a metabolite, a variant and a salt thereof.

## Patentansprüche

1. Verwendung von Cyclosporin, aufgelöst in DMSO, in einem pharmazeutisch verträglichen Träger zur Herstellung eines Medikaments zur Reduktion von oder zur Vorbeugung gegen neuronale Schäden und/oder zum Auslösen von Immunosuppression.

2. Die Verwendung von Anspruch 1, wobei das Medikament zur Injektion in intraventrikulare Flüssigkeitsräume, in intrathecale cerebrospinale Flüssigkeitsräume, auf intraokularem Weg, auf intravestibulärem Weg, in das Gehirn oder in das Rückenmark hergestellt wird.

3. Die Verwendung gemäß Anspruch 2, wobei das Medikament zur Verabreichung durch intra-okulare Injektion oder durch intravestibuläre Injektion, in oder in die Nähe des Gehirns oder Rückenmarks, in einen Menschen hergestellt wird.

4. Die Verwendung gemäß Anspruch 1, wobei besagtes Medikament zur Verabreichung durch intravenöse, intra-arterielle oder intraparenchymale Injektion in einen Menschen hergestellt wird.

5. Die Verwendung gemäß Anspruch 1, wobei besagtes Medikament zur oralen, rektalen, nasalen oder dermalen Verabreichung in einen Menschen hergestellt wird.

6. Die Verwendung gemäß Anspruch 1 bis 5, wobei besagtes Cyclosporin ausgewählt ist aus der Gruppe bestehend aus Cyclosprin A, einem funktionellen Derivat, einem Metaboliten, einer Variante und einem Salz davon.

## Revendications

1. Utilisation d'une cyclosporine dissoute dans le DMSO, dans un support pharmaceutiquement acceptable pour la préparation d'un médicament destiné à réduire ou prévenir un dommage neuronal et/ou à provoquer une immunodépression.

2. Utilisation selon la revendication 1, dans laquelle le médicament est préparé pour être injecté dans les espaces contenant du liquide intraventriculaire, dans les espaces contenant du liquide céphalorachidien intrathécaux, par voie intraoculaire, par voie intravestibulaire, dans le cerveau ou dans la moelle épinière.

3. Utilisation selon la revendication 2, dans laquelle le médicament est préparé pour être administré par injection intraoculaire ou par injection intravestibulaire dans le cerveau ou la moelle épinière à un humain, ou au voisinage de ceux-ci.

4. Utilisation selon la revendication 1, dans laquelle le médicament est préparé pour être administré par injection intraveineuse, intra-artérielle ou intraparenchymale à un humain.

5. Utilisation selon la revendication 1, dans laquelle le médicament est préparé pour être administré par voie orale, rectale, nasale ou dermique à un humain.

6. Utilisation selon les revendications 1 à 5, dans laquelle ladite cyclosporine est choisie dans l'ensemble constitué par la cyclosporine A, un dérivé fonctionnel, un métabolite, un variant et un sel de celle-ci.
